# EUROPÄISCHE PATENTSCHRIFT

(11) **EP 1 261 702 B1**
(45) Veröffentlichungstag und Bekanntmachung des Hinweises auf die Patenterteilung: **21.11.2007**
(21) Anmeldenummer: 01916994.5
(22) Anmeldetag: 14.02.2001
(51) Int. Cl.: C12N 15/10

(54) **VERFAHREN ZUR ABREICHERUNG VON ENDOTOXINEN**
METHOD FOR REMOVING ENDOTOXINS
PROCEDE D'ELIMINATION D'ENDOTOXINES

(30) Priorität: 06.03.2000 DE 10010342
(43) Veröffentlichungstag der Anmeldung: 04.12.2002
(73) Patentinhaber: Merck Patent GmbH, 64293 Darmstadt (DE)
(72) Erfinder: HENDRIKS, Robertus, 69121 Heidelberg (DE); WEHSLING, Maria, 64291 Darmstadt (DE); LANTOS, Andrea, 65929 Frankfurt (DE)
(86) Internationale Anmeldenummer: PCT/EP2001/001610
(87) Internationale Veröffentlichungsnummer: WO 2001/066718

(56) Entgegenhaltungen:
- WO-A-99/63076
- US-A- 5 837 520

## Beschreibung

Die Erfindung betrifft ein Verfahren zur Abreicherung oder Entfernung von Endotoxinen aus biologischem Material. Mittels des erfindungsgemäßen Verfahrens kann beispielsweise hochreine Plasmid-DNA aus natürlichen Quellen gewonnen werden.

Der Bedarf an schnellen und effizienten Verfahren zur Gewinnung von hochreiner Plasmid-DNA aus biologischen Quellen nimmt aufgrund der zunehmenden Bedeutung von rekombinanter DNA für die exogene Expression oder therapeutische Anwendungen ständig zu. Vor allem steigt auch die Nachfrage nach Aufreinigungsmethoden, die auch in größerem Maßstab durchgeführt werden können.

Nahezu alle bekannten Methoden zur Aufreinigung vor allem größerer Mengen an Plasmid-DNA beinhalten einen chromatographischen Aufreinigungsschritt. Die Effizienz dieses Schritts bestimmt in der Regel auch Effizienz und Wirksamkeit der Aufreinigung.

Plasmide sind epigenomische zirkuläre DNA-Moleküle mit einer Länge zwischen 4 und 20 kB, was einem Molekulargewicht zwischen 2,6x10⁶ und 13,2x10⁶ Dalton entspricht. Auch in ihrer kompakten Form (super coil) sind Plasmid-DNA Moleküle normalerweise einige hundert nm groß. Aufgrund dieser Ausmaße sind sie größer als die Poren der meisten Chromatographiematerialien. Dies wiederum bewirkt u.a. die schlechten Bindungskapazitäten der allgemein verwendeten Trennmaterialien für Plasmid-DNA.

Ein weiteres Problem bei der Aufreinigung von Plasmid-DNA stellen die Verunreinigungen dar, von denen die Plasmid-DNA abgetrennt werden soll. Dies ist zum einen genomische DNA und RNA. Diese Moleküle besitzen genau wie Plasmid-DNA einen stark anionischen Charakter und somit ein sehr ähnliches Bindungsverhalten an Trennmaterialien.

Zumindest ebenso aufwendig ist die Abtrennung von Endotoxinen. Endotoxine sind Lipopolysaccharide (LPS), die sich auf der äußeren Membran von Gram-negativen Wirtszellen, wie beispielsweise *Escherichia coli,* befinden. Während der Lyse der Zellen werden neben der Plasmid-DNA auch LPS und andere Membranbestandteile herausgelöst. Endotoxine sind in Zellen in einer Anzahl von ungefähr 3,5x10⁶ Kopien pro Zelle vorhanden (Eschericia Coli and Salmonella Typhimurium Cell. and Mol. Biology, J. L. Ingraham, et al. Eds 1987 ASM) und übertreffen so die Anzahl der Plasmid-DNA Moleküle um mehr als das 10⁴-fache. Aus diesem Grund enthält Plasmid-DNA, die aus Gram-negativen Wirtszellen gewonnen wurde, oft große Mengen an Endotoxinen. Diese Stoffe führen jedoch zu einer Reihe unerwünschter Nebenreaktionen (Morrison and Ryan, 1987, Annu. Rev. Med. 38, 417-432; Boyle et al. 1998, DNA and Cell Biology, 17, 343-348). Soll die Plasmid-DNA beispielsweise zur Gentherapie eingesetzt werden, so ist von größter Bedeutung, daß keine z.B. inflammatorischen oder nekrotischen Nebenreaktionen aufgrund der Verunreinigungen auftreten. Daher besteht ein großer Bedarf an effektiven Verfahren zur Abreicherung von Endotoxinen auf möglichst geringe Mengen.

Bekannte Methoden zur Abreicherung von Endotoxinen basieren auf mehreren Aufreinigungsschritten, häufig unter Einsatz von Silica-Trägern, Glaspulver oder Hydroxylapatit, sowie auf Umkehrphasen-, Affinitäts-, Size-Exclusion- und/oder Anionenaustauschchromatographie.

Zunächst werden die Wirtszellen mittels bekannter Methoden, wie beispielsweise der alkalischen Lyse, aufgeschlossen. Aber auch andere Lyseverfahren, wie z.B. die Anwendung von hohem Druck, die Boiling Lyse, die Verwendung von Detergenzien oder der Aufschluß durch Lysozym sind bekannt.

Die Plasmid-DNA in dem so erhaltenen Medium, einem "Cleared Lysate", ist hauptsächlich durch kleinere Zellbestandteile, Chemikalien aus den vorangegangenen Behandlungsschritten, RNA, Proteine und Endotoxine verunreinigt. Die Abtrennung dieser Verunreinigungen benötigt zumeist mehrere nachfolgende Aufreinigungsschritte. Als besonders vorteilhaft hat sich die Aufreinigung mittels Anionenaustauschchromatographie erwiesen.

Die dynamische Bindungskapazität der meisten Anionentauscher für Plasmid-DNA liegt jedoch nur bei ca. 0,4 mg/ml Trennmaterial. Grund für diesen niedrigen Wert ist, daß die funktionellen Gruppen direkt oder über kurze Spacer an den Träger gebunden sind und so für Wechselwirkungen mit den großen Plasmid-DNA Molekülen nur bedingt zur Verfügung stehen.

Ein weiterer Nachteil der konventionellen Anionenaustauschchromatographie ist, daß zusammen mit der Plasmid-DNA eine beträchtliche Menge an Endotoxinen gebunden wird, die sich auf diese Weise nicht abtrennen läßt. Man erhält Plasmid-DNA mit Endotoxinanteilen von mehr als 500 EU/mg Plasmid-DNA. Zur Abreicherung der Endotoxine sind deshalb weitere Aufreinigungsschritte, wie z.B. chromatographische Schritte (Gelfiltration) oder Fällungen mit Isopropanol, Ammoniumacetat oder Polyethylenglycol, notwendig. Mit Aufreinigungsmethoden, die chromatographische Verfahren, wie z.B. Anionenaustauschchromatographie, und zusätzliche Endotoxinabreicherungsschritte kombinieren, kann man Plasmid-DNA mit einem Endotoxingehalt von unter 50 EU/mg Plasmid DNA erhalten. Allerdings sind derartige Verfahren zumeist aufwendig, zeitintensiv und für die Aufreinigung von größeren Mengen an DNA nur bedingt geeignet.

WO 95/21179 beschreibt ein Verfahren zur Abreicherung von Endotoxinen, bei dem ein Cleared Lysate zunächst mit einer wäßrigen Salzlösung und Detergenzien vorinkubiert wird. Anschließend erfolgt eine Aufreinigung mittels lonenaustauschchromatographie, wobei mit einer weiteren Salzlösung gewaschen, die Plasmid-DNA eluiert wird und anschließend beispielsweise durch Isopropanol-Fällung weiter aufgereinigt wird. Auch dieses Verfahren weist die oben genannten Nachteile auf.

In WO 99/63076 wird statt eines rein anionenaustauschchromatographischen Schritts ein "mixed-mode" Prinzip angewendet, wobei dem Wasch-Puffer 25 bis 90% eines Alkohols zugesetzt werden. Auch dieses Verfahren benötigt typischerweise mehrere Verfahrensschritte, um eine effektive Aufreinigung zu erzielen.

Aufgabe der vorliegenden Erfindung war es daher, ein Verfahren zur chromatographischen Aufreinigung von Plasmid-DNA bereitzustellen, das zum einen ohne weitere Aufreinigungsschritte Plasmid-DNA mit einem Endotoxingehalt von unter 50 EU/mg Plasmid DNA liefert, und das zum anderen auch zur Aufreinigung großer DNA-Mengen geeignet ist.

Es wurde gefunden, daß Plasmid-DNA ohne weitere Fällungsschritte in sehr guter Reinheit erhalten wird, wenn ein Cleared Lysate mit Salz-freier Detergenzlösung vorinkubiert wird und anschließend mittels Anionenaustauschchromatographie auf einem Tentakel-Träger aufgereinigt wird. Das erfindungsgemäße Verfahren ist zur Aufreinigung von kleinen, aber besonders auch zur Aufreinigung von großen Mengen an Plasmid-DNA geeignet.

Im Gegensatz zu der Offenbarung der WO 95/21179, bei der die Vorinkubation mit einer Salz-haltigen Detergenzlösung durchgeführt wird, wurde nun gefunden, daß die Verwendung einer Salz-freien Detergenzlösung, besonders in Kombination mit einer nachfolgenden Anionenaustauschchromatographie an Tentakel-Trägern, eine sehr effiziente Abreicherung von Endotoxinen bewirkt.

Gegenstand der vorliegenden Erfindung ist daher ein Verfahren zur Abreicherung von Endotoxinen aus Nukleinsäuren, die aus natürlichen, gen- oder biotechnologischen Quellen stammen, gekennzeichnet durch folgende Verfahrensschritte:
a) Bereitstellen eines Mediums, das die aufzureinigenden Nukleinsäuren enthält;
b) Vorinkubation des Mediums aus Schritt a) mit einer Salz-freien Detergenzlösung;
c) Aufgabe der Inkubationslösung aus Schritt b) auf Anionenaustauschermaterial, dessen funktionelle Gruppen an Tentakeln auf der Oberfläche des Trägers gebunden sind;
d) Waschen des Anionentauschers, wobei die Verunreinigungen durch Erhöhung der Ionenstärke und/oder pH-Änderungen, d.h. unter anionenaustauschchromatographischen Bedingungen ausgewaschen werden;
e) Elution der Probe durch weitere Erhöhung der Ionenstärke und/oder pH-Änderung.

In einer bevorzugten Ausführungsform enthält die aufzureinigende biologische Quelle Plasmid-DNA.

In einer bevorzugten Ausführungsform des Verfahrens wird ein DEAE oder TMAE Anionenaustauscher verwendet.

In einer bevorzugten Ausführungsform enthält der für Schritt d) verwendete Wasch-Puffer 0 bis 20 % (v/v) eines organischen Lösungsmittels oder Lösungsmittelgemisches, insbesondere eines oder mehrerer Alkhohole, bevorzugt C1 bis C5 Alkohole, besonders bevorzugt Ethanol und/oder Isopropanol.

In einer weiteren bevorzugten Ausführungsform wird das in Schritt a) bereitgestellte Medium durch Aufschlußverfahren, wie alkalischer Lyse, Zentrifugation, Filtration oder Fällung, aus der natürlichen Quelle gewonnen.

Typischerweise ist das Medium ein Cleared Lysate.

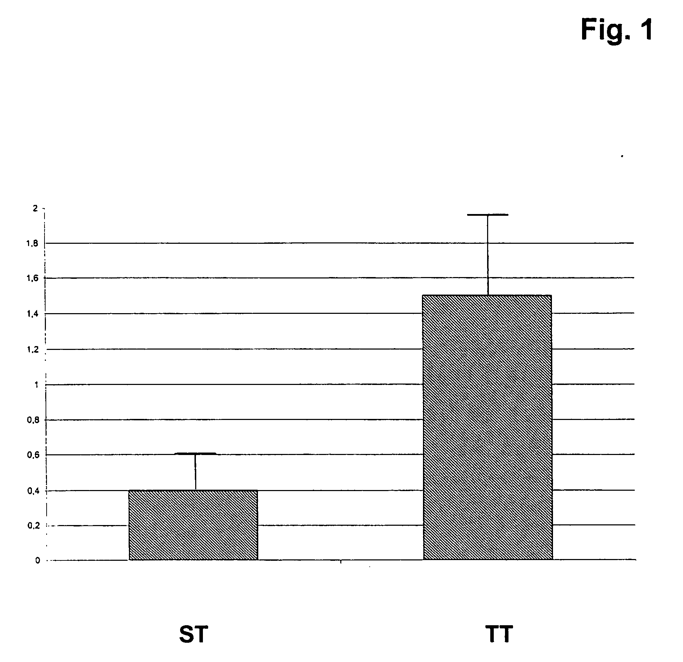

Abbildung 1 zeigt einen Vergleich der dynamischen Bindungskapazitäten von einem Fractogel^{®} EMD TMAE HiCap Tentakel-Träger (TT) mit einem herkömmlichen Silika-DEAE Träger (ST) entsprechend dem in WO 95/21179 verwendeten Material.

Trennmaterialien für die Chromatographie bestehen, wie dem Fachmann bekannt, aus organischen oder anorganischen polymeren Formkörpern. Unter Formkörpern werden erfindungsgemäß poröse und unporöse polymere Materialien, wie beispielsweise perlförmige Formkörper, Membranen, Schläuche, Hohlfasermembranen oder Schwämme verstanden.

Tentakel-Träger im Sinne der vorliegenden Erfindung sind Trennmaterialien, bei denen die funktionellen Gruppen nicht einzeln über Spacer an den Träger gebunden sind. Vielmehr sind die funktionellen Gruppen der erfindungsgemäß eingesetzten Trennmaterialien an Monomereinheiten von Polymerketten gebunden, die auf einen Basisträger aufpolymerisiert sind. Diese flexiblen Polymerstrukturen werden mitunter als "tentakelartig" bezeichnet. Beispiele für Tentakel-Träger werden in WO 96/22316, WO 97/49754, EP 0 337 144, DE 43 34 359 oder WO 95/09695 offenbart. Basismaterialien und funktionelle Gruppen der Träger können je nach der speziellen chromatographischen Fragestellung ausgewählt werden. Bevorzugt werden Tentakel-Träger aus Copolymeren auf Methacrylatbasis eingesetzt, besonders bevorzugt ist dies beispielsweise Fractogel^{®} EMD der Firma Merck KGaA, Deutschland.

Tentakel-Träger zeigen sehr viel höhere Bindungskapazitäten für Plasmid-DNA als herkömmliche Trennmaterialien. Beispiel 1 bzw. Abbildung 1 zeigt einen Vergleich der dynamischen Bindungskapazitäten von einem Fractogel^{®} EMD Tentakel-Träger mit einem Silika-Träger (wie er in WO 95/21179 verwendet wird). Die Bindungskapazität des Fractogel^{®} EMD Tentakel-Trägers liegt bei über 1 mg Plasmid/ml Träger, wohingegen der Silika-Träger nur 0,4 mg Plasmid/ml Träger bindet.

Chromatographische Trennmaterialien bzw. Sorbentien, die zur Auftrennung oder Reinigung von Biopolymeren eingesetzt werden, müssen neben guten Trenneigenschaften eine hohe Alkalistabilität aufweisen. Grund dafür sind insbesondere spezielle Reinigungs- und Sterilisationsverfahren, denen die Sorbentien unterworfen werden.

Beispielsweise wird bei dem sogenannten clean in place Verfahren das Trennmaterial in Abhängigkeit von der Säulendimension über einen Zeitraum von 10 Minuten bis zu drei Stunden mit 1 M Natronlauge behandelt. Um eine Verkeimung zu verhindern, werden die Trennmaterialien für eine Langzeitlagerung in 0,1 M Natronlauge aufbewahrt. Nicht alle Trennmaterialien, die zur Auftrennung von Biopolymeren eingesetzt werden, sind unter derartigen Bedingungen stabil.

Basisträger für die Biochromatographie, wie sie auch für den erfindungsgemäßen Einsatz z.B. in EP 0 337 144 oder WO 95/09695 offenbart werden, sind beispielsweise natürliche Polymere, wie Dextran, Agarose oder Cellulose, Kieselgel oder synthetische Polymere, wie Polystyrol und Methacrylatester. Vor allem Trennmaterialien auf Basis von Kieselgel zeigen bekanntermaßen eine schlechte Alkalistabilität. Doch auch Träger auf Basis von Methacrylatestern können nur bedingt einer Alkalibehandlung unterzogen werden. Für den erfindungsgemäßen Einsatz werden daher besonders Trennmaterialien bevorzugt, die eine hohe Alkalistabilität aufweisen. Auf diese Weise können die Trennmaterialien für den Einsatz in der Biochromatographie ausreichend sterilisiert werden und eigenen sich sogar für den mehrmaligen Gebrauch.

Besonders bevorzugt für das erfindungsgemäßeverfahren sind daher alkalistabile Sorbentien, die hergestellt werden durch Umsetzung eines Tentakel-Trägers, der reaktive Gruppen der Formel I aufweist, mit einer Verbindung der Formel II.

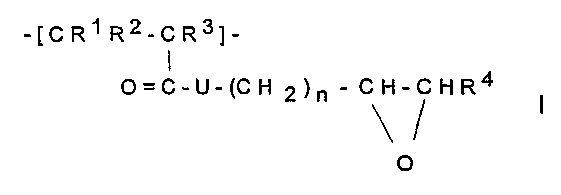

In Formel I bedeuten:
- R¹,R² und R³: unabhängig voneinander
H oder CH₃,
- R₄: H, Alkyl mit 1-5 C-Atomen oder Aryl mit 6-12 C-Atomen,
- U: -O- oder -NH-
und
- n: eine ganze Zahl zwischen 1 und 5.

A-(CH₂)ₘ-B II

In Formel II bedeuten
- A: NHR⁵,
- B: H, OH, SH oder NHR⁵ mit
- R⁵: H, Alkyl mit 1-5 C-Atomen, bevorzugt H, Methyl oder Ethyl
und
- m: eine ganze Zahl zwischen 1 und 6, bevorzugt 2 und 3.

Besonders bevorzugt weist der Träger reaktive Gruppen der Formel I auf, in denen
- R¹ und R²: H,
- R3: CH₃,
- R4: H,
- U: -O
und
- n: 1
bedeuten. Diese Gruppen entstehen vorzugsweise durch Block- oder Pfropfpolymerisation von Glycidylmethacrylat auf ein Basispolymer.

Besonders bevorzugt wird Ethanolamin als Verbindung der Formel II verwendet.

Ganz besonders bevorzugt besteht der Träger aus einem Basispolymer aus Polyamid, Polyvinylalkohol oder Copolymeren aus (Meth)acrylatderivaten und Comonomeren mit aliphatischen Hydroxylgruppen.

Der Träger kann zusätzlich Gruppen der Formel III aufweisen, die Separationseffektoren tragen.

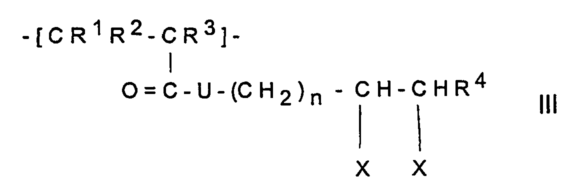

In Formel III bedeuten:
- R¹,R² und R³: unabhängig voneinander
H oder CH₃,
- R₄: H, Alkyl mit 1-5 C-Atomen oder Aryl mit 6-12 C-Atomen,
- U: -O- oder -NH-
- n: eine ganzen Zahl zwischen 1 und 5,
ein Rest X einen Separationseffektor, sowie der andere Rest X OH.

Der Separationseffektor stellt insbesondere eine ionische Gruppe, ausgewählt aus -NR⁷R⁸ oder -N+R⁷R⁸R⁹ dar,
worin
- R⁷ und R⁸: unabhängig voneinander
H, Alkyl mit 1-5 C-Atomen
und
- R₉: Alkyl mit 1-5 C-Atomen
mit der Maßgabe, daß wenn X = -N+R⁷R⁸R⁹, R⁷ und R⁸ nicht H sein können.

Die Stabilisierung der Träger, die reaktive Gruppen entsprechend Formel I enthalten, erfolgt durch Umsetzung mit einer Verbindung entsprechend Formel II. Typischerweise wird der Träger dazu mit einer wässrigen 0,5 bis 5 M Lösung der entsprechenden Verbindung versetzt und bei 20 bis 60°C über 1 bis 6 Stunden umgepumpt. Besonders bevorzugt ist eine Umsetzung mit Verbindungen der Formel II, in denen m gleich 1 oder 2 ist. Längerkettige Verbindungen erhöhen den hydrophoben Charakter des Formkörpers.

Durch diesen abschließenden Stabilisierungsschritt werden die im allgemeinen basenlabilen Polymere oder aufpolymerisierten Schichten aus Acrylat-Derivaten wesentlich stabiler gegenüber einer Alkalibehandlung. Somit wird ein wesentlicher Nachteil der durch Block- oder Propfpolymerisation mit Acrylatestern hergestellten Sorbentien, die sich ansonsten durch sehr gute Bindungskapazitäten auszeichnen, behoben.

Bevorzugte funktionelle Gruppen für den erfindungsgemäßen Einsatz sind Trimethylammoniumethyl (TMAE), Diethylaminoethyl (DEAE), oder Dimethylaminoethyl (DMAE).

Zusätzlich zu den hohen Bindungskapazitäten und der Stabilität der Tentakel-Träger wurde nun gefunden, daß sie besondere Vorteile bei der Aufreinigung von Plasmid-DNA mittels Anionenaustauschchromatographie aufweisen. Durch das erfindungsgemäße Verfahren unter Verwendung der Tentakel-Träger kann der Gehalt an Endotoxinen gegenüber Standardmethoden um das 10 bis 20-fache reduziert werden. Weitere Reinigungsschritte, die im Stand der Technik im Anschluß an die chromatographische Reinigung durchgeführt werden müssen, können deswegen bei dem erfindungsgemäßen Verfahren in der Regel entfallen.

Das erfindungsgemäße Verfahren eignet sich besonders zur Aufreinigung von Nukleinsäuren. Dies sind einzelsträngige oder doppelsträngige RNA oder DNA, RNA/DNA Hybride, DNA-Fragmente, Oligonukleotide, amplifizierte DNA oder RNA, BACs, oder vor allem Plasmid-DNA. Die Größe der Nukleinsäuren kann zwischen 6 b/bp und 1000 kb/kbp liegen.

Die aufzureinigenden Nukleinsäuren können aus jeder natürlichen, gen- oder biotechnologischen, Quelle, wie beispielsweise prokaryotischen Zellkulturen, stammen. Falls Nukleinsäuren aus Zellpräparaten aufgereinigt werden sollen, werden die Zellen zunächst nach bekannten Methoden, wie beispielsweise Lyse, aufgeschlossen. Falls das aufzureinigende Material bereits auf andere Weise vorbehandelt wurde, kann ein lytischer Aufschluß entfallen. Beispielsweise kann das Medium aus biologischem Material durch Entfernung der Zelltrümmer und eines Niederschlags von RNA gewonnen werden, aus Nukleinsäure-Proben, die bereits vorgereinigt sind und z.B. in Puffer vorliegen oder auch aus Nukleinsäurelösungen, die nach einer Amplifikation entstanden sind und noch Verunreinigungen durch Endotoxine aufweisen. Gegebenenfalls sind Filtration, Fällungs-oder Zentrifugationsschritte notwendig. Der Fachmann ist in der Lage, in Abhängigkeit von der Quelle des biologischen Materials ein geeignetes Aufschlußverfahren zu wählen. In jedem Fall sollte die aufzureinigende Probe für das erfindungsgemäße Verfahren in einem Medium vorliegen, das bei Zugabe der Detergenzlösung keine Niederschläge bildet oder sonstige unerwünschte Nebenreaktionen hervorruft. Bevorzugt handelt es sich bei dem Medium um ein Lysat, das aus Zellen gewonnen wurde, wie z.B. ein Cleared Lysate.

Zur Aufreinigung von Plasmid-DNA aus *E.coli* werden die Zellen beispielsweise zunächst durch alkalische Lyse mit NaOH/SDS-Lösung lysiert. Durch Zugabe eines sauren Kalium-haltigen Neutralisationspuffers bildet sich dann ein Präzipitat, das durch Zentrifugation oder Filtration entfernt werden kann. Der verbleibende, klare Überstand, das Cleared Lysate, kann nun als Ausgangsmaterial, d.h. als Medium, für das erfindungsgemäße Verfahren eingesetzt werden. Es ist auch möglich, das Cleared Lysate zunächst nach bekannten Methoden, wie Dialyse oder Präzipitation, aufzukonzentrieren oder vorzureinigen.

Im ersten Schritt des erfindungsgemäßen Verfahrens wird das Cleared Lysate nun mit einer Salz-freien, nicht ionischen Detergenz-Lösung versetzt. Falls durch Verunreinigungen der Detergenzien Salze in die Lösung gelangen, so sollte deren Konzentration unter 0,005%, d.h. unter 1 mM betragen. Als Detergenzien werden nicht-ionische Detergenzien, wie beispielsweise Triton^{®} X-100, Triton^{®} X-114, Tween^{®} 20 oder Igepal^{®} CA 630 oder Mischungen davon eingesetzt. Die Detergenzien liegen vorzugsweise nach der Zugabe zu der Probe in Endkonzentrationen zwischen 0,01 und 30% (v/v), besonders bevorzugt zwischen 0,5 und 15% (v/v) vor.

Nach der Vorinkubation wird das Lysat auf einen erfindungsgemäß geeigneten Anionenaustauscher gegeben. Beladung, Waschen und Elution erfolgt nach bekannten Verfahren. Ausmaß und Stärke der Bindung eines Ziel-Moleküls an den Ionenaustauscher hängt u.a. von pH und Ionenstärke des Puffers, dem isoelektrischen Punkt des Target-Moleküls und der Dichte der Ladungen auf der Matrix ab. Generell wird die Probe auf eine mit einem Puffer niedriger Ionenstärke äquilibrierte Säule geladen, wonach ungebundene Molekülen ausgewaschen werden. Durch Erhöhung der Ionenstärke und/oder pH Änderung werden Verunreinigungen selektiv ausgewaschen, bevor durch weitere Erhöhung der Ionenstärke und/oder Veränderung des pH die Ziel-Moleküle eluiert werden.

Der Fachmann ist in der Lage, für den jeweiligen Tentakel-Träger bzw. das zu reinigende biologische Material geeignete Reagenzien zu wählen. Es ist dabei zu beachten, daß die Wechselwirkung zwischen der Plasmid-DNA und der Anionenaustauschgruppe eines z.B. Fractogel^{®} EMD TMAE Trägers stärker von Unterschieden in der Ionenstärke beeinflußt wird als dies bei der Verwendung herkömmlicher Träger, z.B. Silika-DEAE Trägem, der Fall ist. Obwohl der pK von TMAE mit über 13 höher ist als der von DEAE mit 11, müssen bei der Verwendung von Fractogel^{®} EMD Tentakel Trägern daher spezielle Beladungsbedingungen eingehalten werden. Besonders ist darauf zu achten, daß die Ionenstärke der Probe, mit der der Träger beladen werden soll, weit genug unterhalb der des Elutionspunktes der Plasmid-DNA liegt.

Es hat sich z.B. gezeigt, daß die Verwendung von isopropanolhaltigen Puffern anstelle von ethanolhaltigen Puffern vorteilhaft ist. Wie in DE 44 03 693 vorgeschlagen, können durch die Verwendung von isopropanolhaltigen Puffern besonders gute Transfektionsraten (bzw. geringere Endotoxin Verunreinigung) der hergestellten Nukleinsäuren erzielt werden. Die erfindungsgemäß eingesetzten Wasch- und Elutionspuffer sollten nicht mehr als 20% (v/v) eines oder mehrerer organischer Lösungsmittel enthalten, damit das Auswaschen der Verunreinigungen und auch die Elution der Nukleinsäuren anionenaustauschchromatographisch, d.h. vor allem in Abhängigkeit der Ionenstärke und/oder von pH Änderungen, erfolgt und keine weiteren Waschschritte erforderlich sind. Insbesondere werden als organische Lösungsmittel C1 bis C5- Alkohole, bevorzugt Ethanol und/oder Isopropanol eingesetzt. Besonders bevorzugt enthalten die Puffer zwischen 10 und 15 % Ethanol und/oder Isopropanol.

Somit beinhaltet das erfindungsgemäße Verfahren typischerweise die folgenden Schritte:
- Inkubation des Mediums, das die Nukleinsäuren enthält, (typischerweise das Cleared Lysate) mit einer Salz-freien Detergenzienlösung (Dauer ca. 15-30 min)
- Aufgabe der Inkubationslösung auf die vorkalibrierte Anionenaustauschersäule
- Waschen der Säule mit einem Waschpuffer
- Elution der Plasmid-DNA mit einem Elutionspuffer

Nach der Elution von dem Anionenaustauscher weist die nach dem erfindungsgemäßen Verfahren aufgereinigte Plasmid-DNA einen Endotoxin-Gehalt von unter 50 EU/mg Plasmid, zumeist sogar einen Endotoxin-Gehalt von unter 25 EU/mg Plasmid auf. Diese Werte werden in herkömmlichen Verfahren nur durch zusätzliche Reinigungsschritte, wie Präzipitation oder Gelfiltration, erreicht.

Damit ist das erfindungsgemäße Verfahren, das eine Salz-freie Vorinkubation mit einem Chromatographieschritt auf einem speziellen Anionentauscher kombiniert, 5 bis 10 mal effizienter als andere Verfahren, die beispielsweise zur Vorinkubation eine Salz-haltige Detgergenzlösung verwenden. Wird die Vorinkubation des erfindungsgemäßen Verfahrens mit einer Salz-haltigen Detergenzlösung durchgeführt, sinkt die Effizienz der Aufreinigung. Demnach führt eine Steigerung der Ionenstärke während der Vorinkubation bei der Verwendung von Tentakel-Trägern, wie z.B.

Fractogel^{®} EMD Anionentauschern, zu einer Erhöhung des Endotoxingehaltes der eluierten Probe.

Die eluierten Nukleinsäuren können gesammelt oder fraktioniert aufgefangen werden und ihrer weiteren Verwendung zugeführt werden. Da die aufgereinigten Proben nach der Elution von dem Anionentauscher zumeist durch die Salze des Elutionspuffers verunreinigt sind, kann es notwendig sein, die Probe zur Entfernung der Salze zusätzlich z.B. mittels Dialyse oder Alkoholfällung aufzureinigen. Diese Schritte dienen jedoch bei dem erfindungsgemäßen Verfahren lediglich zur Entfernung von Salzen, nicht aber, wie im Stand der Technik, zur weiteren Abreicherung von Endotoxinen.

Neben der Qualität der aufgereinigten Nucleinsäuren, ist auch der Maßstab in dem die Nucleinsäuren aufgereinigt werden können, von entscheidender Bedeutung. Das erfindungsgemäße Verfahren ist sowohl zur Aufreinigung kleiner Mengen (unter 1 mg), wie auch zur Aufreinigung großer Mengen (von 1 mg bis zu einigen 100 g und darüber hinaus) geeignet.

### Beispiele

Für die Aufreinigungen wurden folgende Lösungen verwendet:
1. CL Resuspensions Puffer
   50 mM Tris.HCl, pH 8.0
   10 mM EDTA
   100 µg/ml DNAse-freie Ribonuklease A
2.CL Lyse Puffer
   200 mM NaOH
   1 % (w/v) SDS (Natrium Dodecyl Sulphat)
3.CL Neutralisations-Puffer
   3.0 M KOAc (Kaliumacetat), pH 5.5
4.IE Equilibrations-Puffer
   50 mM Tris, pH 6.5
   500 mM NaCl
   15% (v/v) Ethanol
5.IE Wasch-Puffer
   50 mM Tris, pH 6.5
   625 mM NaCl
   15% (v/v) Ethanol
6.IE Elutions-Puffer
   50 mM Tris, pH 8.5
   1500 mM NaCl
   15% (v/v) Ethanol

Die Bakterienkulturen wurden nach bekannten Methoden kultiviert und zu einem Cleared Lysate verarbeitet. Daher wird dies im folgenden nur kurz erläutert:
1. Zellen aus einer 50 ml bis zu 3 l Bakterienkultur wurden 15 min bei 6000 g zentrifugiert und in 4 ml CL Resuspensions-Puffer pro 50 ml Kultur resuspendiert. Man erhält eine trübe Suspension, die frei von größeren Zellklumpen ist.
2. Man fügt das gleiche Volumen CL Lyse Puffer hinzu und mischt ("Überkopf drehen"/ wirbeln / schwenken) bis die Lösung klar ist. Diese Lösung darf nicht länger als 10 Minuten bei Raumtemperatur gelagert werden.
3. Man fügt dann das gleiche Volumen CL Neutralisations-Puffer hinzu (4 ml pro 50 ml Kultur) und mischt bis das Lysat trübe wird und ausflockt. Anschließend wird das Lysat 15-20 Minuten auf Eis inkubiert.
4. Die Lösung wird dann 30 min bei 4 °C mit über 20.000 g zentrifugiert, um ein klares Lysat zu erzeugen.
5. Der Überstand des "Cleared Lysate" wird in ein neues Behältnis überführt.

### Beispiel 1: Vergleich der Bindungskapazitäten

Die dynamischen Bindungskapazitäten von einem Fractogel^{®} EMD TMAE Tentakel-Träger (TT) mit einem herkömmlichen Silika-Träger (ST) der Firma Qiagen mit aufgepfropften funktionellen Gruppen wurden verglichen.

Bakterien (Übernacht-Kulturen DH5α transformiert mit pTriEx Plasmid, JM109 transformiert mit pBluescipt Plasmid und NovaBlue transformiert mit pTriEx Plasmid) wurden pelletiert und nacheinander mit CL Resuspensions-, Lyse und Neutralisations-Puffer resuspendiert, lysiert und neutralisiert. Die Zelltrümmer der verschiedenen Lysate wurden abzentrifugiert.
An dieser Stelle wurden die verschiedene Cleared Lysate je in zwei Fraktionen aufgeteilt, um sicherzustellen, daß für die Aufreinigungen identisches Ausgangsmaterial verwendet wurde.

Eine der beide Fraktionen von jedem Cleared Lysat (alle mit sättigender Menge an Plasmid im Bezug auf die Trägermenge) wurde auf den Fractogel^{®} Träger geladen, die andere auf den Silika-Träger.

Die Fractogel^{®} EMD TMAE HiCap Säulen wurden vor der Beladung mit den Proben mit 5 Säulenvolumina des IE Equilibrations-Puffers äqulibriert. Zur Anionenaustauschchromatographie dem Silika-Träger QIAGEN^{®} Plasmid Maxi Kit, Cat No. 12163 wurden die in WO 95/21179, Beispiel 1, genannten Puffer verwendet. Während der Beladung der Säulen wurden alle Eluate in Fraktionen gesammelt und auf ihren Plasmid-Gehalt hin analysiert.

Abbildung 1 zeigt das Ergebnis der Untersuchung. Es wurden die Ergebnisse von 4 unabhängigen Versuchen verrechnet. Auf der Ordinate ist die dynamische Bindungskapazität in mg Plasmid-DNA/ml Träger angegeben. Es zeigt sich, daß der Fractogel^{®} EMD TMAE Träger eine wesentlich höhere Bindungskapazität für Plasmid-DNA besitzt.

### Beispiel 2: Aufreinigung von pBacMam DNA

Bakterien (eine Übemacht-Kultur von DH5α Zellen, transformiert mit einem pBacMam Plasmid) wurden pelletiert und nacheinander mit CL Resuspensions-, Lyse und Neutralisations-Puffer resuspendiert, lysiert und neutralisiert. Die Zelltrümmer wurden abzentrifugiert.

An dieser Stelle wurde das Cleared Lysate in unterschiedliche Fraktionen aufgeteilt, um sicherzustellen, daß für alle Aufreinigungen identisches Ausgangsmaterial verwendet wird.
Es wurden folgende Fraktionen gebildet:
(a) Das Cleared Lysate wird nicht vorinkubiert, sondern direkt auf den Fractogel^{®} Träger geladen.
(b) Das Cleared Lysate wird 30 min mit 1/10 seines Volumens einer Salz-freien 20% Triton^{®} X-114 Lösung vorinkubiert und dann auf den Fractogel^{®} Träger geladen.
(c) Das Cleared Lysate wird nach bekannten Verfahren 2 bis 3 aufeinanderfolgenden Phasentrennungen mit nicht-kondensierter Triton^{®} X-114 Detergenzlösung unterzogen (Manthorpe et al. (1993), Hum Gene Ther. 4, 419-431) und dann auf den Fractogel^{®} Träger geladen.

Die Fractogel^{®} EMD TMAE HiCap Säulen wurden vor der Beladung mit den Proben mit 5 Säulenvolumina des IE Equilibrations-Puffers äqulibriert. Nach der Beladung wurden die Säulen mit 10 Säulenvolumina des IE Wasch-Puffers gewaschen und mit 4 Säulenvolumina des IE Elutions-Puffers eluiert. Das Eluat wurde ohne weitere Aufreinigungsschritte mittels LAL (limulus amoebocyte lysate) Test analysiert (Bayston and Cohen (1990), J. Med. Microbiol. 31, 73-83). Der Test wurde entsprechend dem Europäischen Arzneibuch, 3. Ausgabe (1997) Kapitel 2.6.14 durchgeführt und nach der FDA-Richtlinie "Guideline on validation of the LAL Test as an endproduct endotoxine test for human and animal parenteral drugs, biological products and medical devices" von Dezember 1987. Die Lysat Sensitivität lag unter 0,12 EU/ml.

Tabelle 1 zeigt die aus drei unabhängigen Experimenten erhaltenen Endotoxinwerte:

**Tabelle 1**

| | **Ohne Vorinkubation** | **Inkubation mit Salz-freier Detergenz-Lösung** | **Triton^{®} X-114 Phasentrennung** |
|---|---|---|---|
| | Endotoxin Kontamination in E.U. / mg pBacMam | | |
| 1 | 116,36 | 7,27 | 8,00 |
| 2 | 53,30 | 5,10 | 6,30 |
| 3 | 147,70 | 5,30 | 4,00 |
| Durchschnitt | **105,79 ± 39,26** | **5,89± 0,98** | **6,10± 1,64** |

Bei Experiment 2 wurden zwei aufeinanderfolgende Triton^{®} X-114 Detergenz Phasentrennungen durchgeführt, bei Experiment 1 und 3 jeweils drei.

Durch Kombination einer Vorinkubation mit Salz-freier Detergenzlösung mit einer Anionenaustauschchromatographie auf einem Fractogel^{®} EMD Anionentauscher erhält man Endotoxinwerte von < 25 EU/mg Plasmid DNA. Ohne weitere Aufreinigungsschritte, wie Fällung oder Gelfiltration, enthält die DNA genausowenig Endotoxine wie nach der sehr viel aufwendigeren Detergenz Phasentrennung.

### Beispiel 3: Aufreinigung von pTriEx DNA unter Einsatz verschiedener nicht-ionischer Detergenzien

Bakterien (eine Obemacht-Kultur von DH5α Zellen, transformiert mit einem pTriEx Plasmid) wurden pelletiert und nacheinander mit CL Resuspensions-, Lyse und Neutralisations-Puffer resuspendiert, lysiert und neutralisiert. Die Zelltrümmer wurden abzentrifugiert.
An dieser Stelle wurde das Cleared Lysate in unterschiedliche Fraktionen aufgeteilt, um sicherzustellen, daß für alle Aufreinigungen identisches Ausgangsmaterial verwendet wird.
Es wurden folgende Fraktionen gebildet:
(a) Das Cleared Lysate wird nicht vorinkubiert, sondern direkt auf den Fractogel^{®} Träger geladen.
(b) Das Cleared Lysate wird 60 min auf Eis mit 1/10 seines Volumens verschiedener Salz-freier 20% Detergenz-Lösung vorinkubiert und dann auf den Fractogel^{®} Träger geladen.
Die Fractogel^{®} EMD TMAE HiCap Säulen wurden vor der Beladung mit den Proben mit 5 Säulenvolumina des IE Equilibrations-Puffers äqulibriert. Nach der Beladung wurden die Säulen mit 10 Säulenvolumina des IE Wasch-Puffers gewaschen und mit 4 Säulenvolumina des IE Elutions-Puffers eluiert. Das Eluat wurde ohne weitere Aufreinigungsschritte mittels turbidimetrischem LAL Test analysiert. Die Ergebnisse sind in Tabelle 2 dargestellt:

**Tabelle 2**

| | **Salz-freie Detergenzlösung** | **Endotoxin Kontamination in E.U. / mg pTriEx** |
|---|---|---|
| 1 | ohne Vorinkubation | 116,36 |
| 2 | Triton^{®} X-100 | 12,63 |
| 3 | Triton^{®} X-114 | 3,58 |
| 4 | Igepal^{®} CA 630 | 4 |
| 5 | Tween^{®} 20 | 14,55 |

Es zeigt sich, daß die Vorinkubation mit Salz-freier Detergenzlösung zu einer signifikanten Reduktion des Endotoxingehaltes führt.

### Beispiel 4: Aufreinigung von pTriEx DNA mit Salz-freien und Salz-haltigen Igepal^{®}-Detergenzlösungen auf Fractogel^{®} EMD TMAE HiCap und QIAGEN^{®} Trägem.

Die Herstellung des Cleared Lysate erfolgte wie in Beispiel 3 beschrieben, jedoch ausgehend von einer Übemacht-Kultur von NovaBlue Zellen, transformiert mit einem pTriEx Plasmid.

Das Cleared Lysate wird in unterschiedliche Fraktionen aufgeteilt, um sicherzustellen, daß für alle Aufreinigungen identisches Ausgangsmaterial verwendet wird.
(a) 2 Fraktionen wurden nicht vorinkubiert, sondern direkt auf die beiden Träger (Fractogel^{®} EMD TMAE HiCap und QIAGEN ^{®}-DEAE) gegeben.
(b) 2 Fraktionen wurden mit einer Salz-haltigen Detergenzlösung vorinkubiert und dann auf die Träger gegeben. Die Vorinkubation erfolgte entsprechend WO 95/21179, Beispiel 1.
(c) 2 Fraktionen wurden mit Salz-freier Detergenzlösung entsprechend Beispiel 3 vorinkubiert und dann auf die Träger gegeben.

Zur Anionenaustauschchromatographie auf dem Träger der Firma Qiagen wurden die in WO 95/21179, Beispiel 1 genannten Puffer verwendet. Da die Elutionspunkte der Plasmid-DNA für die beiden Trennmaterialien unterschiedlich sind, können die Puffer jedoch nicht eine völlig gleiche Zusammensetzung aufweisen. Für den Fractogel^{®} EMD TMAE HiCap Träger wurden die zu Anfang der Beispiele genannten Puffer verwendet.

Die Eluate der Ionenaustauschsäulen wurden gesammelt und direkt,ohne weitere Aufreinigungsschritte mittels LAL-Test auf ihren Endotoxin-Gehalt hin untersucht.
Die Ergebnisse sind in Tabelle 3 und 4 dargestellt:

**Tabelle 3**

| **aufgegebene Menge** | 2211,84 EU gesamt | 2211,84 EU gesamt |
|---|---|---|
| | **Eluat Fractogel^{®}** | **Eluate QIAGEN**^{®} |
| ohne Vorinkubation | 330,7 EU/mg | 981,5 EU/mg |
| Vorinkubation mit Salz-freier Detergenzlösung | 19,5 EU/mg | 458,2 EU/mg |
| Vorinkubation mit Salz-haltiger Detergenzlösung | 83,2 EU/mg | 924,6 EU/mg |

Somit ergeben sich mit den beiden Trägern und der Vorinkubation (mit und ohne Salz) folgende Verbesserungen gegenüber einer lonenaustauschchromatographie ohne Vorinkubation (Standard IEC):

**Tabelle 4**

| | **Fractogel^{®}** | | **QIAGEN^{®}** | |
|---|---|---|---|---|
| | gesamt | *Verbesserung gegenüber* Standard IEC | gesamt | *Verbesserung gegenüber* Standard IEC |
| ohne Vorinkubation | 307,2 EU | / | 921,6 EU | / |
| Vorinkubation mit Salz/Detergenz | 76,8 EU | 4-fach | 921,6 EU | keine |
| Vorinkubation mit Detergenz | 19,2 EU | 16-fach | 460,8 EU | 2-fach (nicht signifikant) |

Die Tabelle enthält die Mittelwerte aus zwei unabhängigen Versuchen. Es zeigt sich, daß die Kombination -Vorinkubation mit Salz-freier Detergenzlösung/Fractogel^{®} Träger- bei weitem die beste Aufreinigung bewirkt.

## Patentansprüche

1. Verfahren zur Abreicherung von Endotoxinen aus Nukleinsäuren, die aus natürlichen, gen- oder biotechnologischen Quellen stammen, **gekennzeichnet durch** folgende Verfahrensschritte:
a) Bereitstellen eines Mediums, das die aufzureinigenden Nukleinsäuren enthält;
b) Vorinkubation des Mediums aus Schritt a) mit einer Salz-freien Detergenzlösung;
c) Aufgabe der Inkubationslösung aus Schritt b) auf Anionenaustauschermaterial, dessen funktionelle Gruppen an Tentakeln auf der Oberfläche des Trägers gebunden sind;
d) Waschen des Anionentauschers, wobei die Verunreinigungen durch Erhöhung der Ionenstärke und/oder pH-Änderungen ausgewaschen werden und wobei der verwendete Wasch-Puffer 0 bis 20 % (v/v) eines organischen Lösungsmittels oder Lösungsmittelgemisches enthält;
e) Elution der Probe **durch** weitere Erhöhung der Ionenstärke und/oder pH-Änderung.

2. Verfahren zur Abreicherung von Endotoxinen nach Anspruch 1, **dadurch gekennzeichnet, daß** die aufzureinigende biologische Quelle Plasmid-DNA enthält.

3. Verfahren nach einem der Ansprüche 1 oder 2, **dadurch gekennzeichnet, daß** der Anionenaustauscher DEAE oder TMAE als funktionelle Gruppen aufweist.

4. Verfahren nach einem der Ansprüche 1 bis 3, **dadurch gekennzeichnet, daß** das in Schritt a) bereitgestellte Medium durch Aufschlußverfahren, wie alkalischer Lyse, Zentrifugation, Filtration oder Fällung, aus der natürlichen Quelle gewonnen wurde.

## Claims

1. Method for reducing endotoxin levels from nucleic acids originating from natural, gene- or biotechnological sources, **characterised by** the following steps:
a) provision of a medium which contains the nucleic acids to be purified;
b) pre-incubation of the medium from step a) with a salt-free detergent solution;
c) application of the incubation solution from step b) to anion-exchanger material whose functional groups are bonded to tentacles on the surface of the support;
d) washing of the anion exchanger, where the impurities are washed out by increasing the ion strength and/or by pH changes and where the wash buffer used comprises 0 to 20% (v/v) of an organic solvent or solvent mixture;
e) elution of the sample by a further increase in the ion strength and/or pH change.

2. Method for reducing endotoxin levels according to Claim 1, **characterised in that** the biological source to be purified comprises plasmid DNA.

3. Method according to one of Claims 1 and 2, **characterised in that** the anion exchanger contains DEAE or TMAE as functional groups.

4. Method according to one of Claims 1 to 3, **characterised in that** the medium provided in step a) has been obtained from the natural source by digestion methods, such as alkaline lysis, centrifugation, filtration or precipitation.

## Revendications

1. Procédé pour réduire des niveaux d'endotoxine dans des acides nucléiques provenant de sources naturelles, génétiques ou biotechnologiques, **caractérisé par** les étapes qui suivent:
a) fourniture d'un milieu qui contient les acides nucléiques à purifier;
b) pré-incubation du milieu issu de l'étape a) avec une solution de détergent exempte de sel;
c) application de la solution d'incubation issue de l'étape b) sur un matériau échangeur d'anions dont des groupes fonctionnels sont liés à des tentacules sur la surface du support;
d) lavage de l'échangeur d'anions, où les impuretés sont évacuées par lavage en augmentant l'intensité ionique et/ou au moyen de modifications de pH et où le tampon de lavage utilisé comprend de 0 à 20% (v/v) d'un solvant organique ou d'un mélange de solvants organiques;
e) élution de l'échantillon au moyen d'une augmentation supplémentaire de l'intensité ionique et/ou de modification de pH.

2. Procédé pour réduire des niveaux d'endotoxine selon la revendication 1, **caractérisé en ce que** la source biologique à purifier comprend de l'ADN plasmidique.

3. Procédé selon l'une des revendications 1 et 2, **caractérisé en ce que** l'échangeur d'anions contient DEAE ou TMAE en tant que groupes fonctionnels.

4. Procédé selon l'une des revendications 1 à 3, **caractérisé en ce que** le milieu fourni au niveau de l'étape a) a été obtenu à partir de la source naturelle au moyen de procédés de digestion, tels qu'une lyse alcaline, une centrifugation, une filtration ou une précipitation.
